(19) Europäisches Patentamt
European Patent Office
Office européen des brevets

(11) Veröffentlichungsnummer : **0 174 464**
**B1**

(12) **EUROPÄISCHE PATENTSCHRIFT**

(45) Veröffentlichungstag der Patentschrift : **03.01.90**

(51) Int. Cl.[5] : **C 07 D403/04**, A 61 K 31/50

(21) Anmeldenummer : **85108760.1**

(22) Anmeldetag : **12.07.85**

(54) **Substituierte Benzylphthalazinon-Derivate.**

(30) Priorität : **14.09.84 DE 3433776**

(43) Veröffentlichungstag der Anmeldung : **19.03.86 Patentblatt 86/12**

(45) Bekanntmachung des Hinweises auf die Patenterteilung : **03.01.90 Patentblatt 90/01**

(84) Benannte Vertragsstaaten : **AT BE CH DE FR GB IT LI LU NL SE**

(56) Entgegenhaltungen :
**DE-A- 2 164 058**
**US-A- 3 813 384**
**Die Akte enthält technische Angaben, die nach dem Eingang der Anmeldung eingereicht wurden und die nicht in dieser Patentschrift enthalten sind.**

(73) Patentinhaber : **ASTA Pharma AG**
**Weismüllerstrasse 45**
**D-6000 Frankfurt am Main 1 (DE)**

(72) Erfinder : **Engel, Jürgen, Dr.**
**Cranachstrasse 25**
**D-8755 Alzenau (DE)**
Erfinder : **Scheffler, Gerhard, Dr.**
**Frankenwaldstrasse 19**
**D-6450 Hanau 6 (DE)**

**EP 0 174 464 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der deutschen Patentschrift 21 64 058 sind basisch substituierte 4-Benzyl-1-(2H)-phthalazinon-Derivate der folgenden Formel

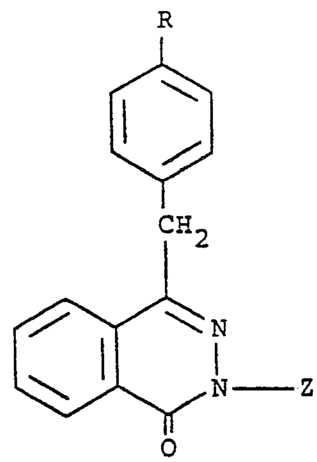

worin R ein Wasserstoff- oder Halogenatom, eine Trifluormethylgruppe oder eine niedere Alkyl- oder Alkoxygruppe und Z einen 4-Perhydroazepinyl-, N-Methyl-4-perhydroazepinyl-, 3-Chinuclidyl-, 3-Tropanyl-, 3-Nortropanyl-, N-Methyl-3-pyrrolidinyl- oder N-Methyl-2-pyrrolidinyl-methyl-Rest bedeutet, sowie die physiologisch unbedenklichen Säureadditionssalze hiervon.

Diese Verbindungen besitzen eine Antihistamin-Wirkung. Die Erfindung betrifft die durch die Patentansprüche definierten Gegenstände.

Die erfindungsgemäßen Verbindungen sind antiallergisch und asthmaprophylaktisch wirksam, jedoch erheblich stärker und besser als die vorbekannten Verbindungen der deutschen Patentschrift 21 64 058. Darüberhinaus besitzen sie im Gegensatz zu dem bekannten Arzneimittelwirkstoff AZELASTIN (Verbindung gemäß Beispiel 5 der deutschen Patentschrift 21 64 058) keinen, beziehungsweise einen erheblich geringeren bitteren Geschmack, so daß sie ohne weiteres beispielsweise auch als Aerosol appliziert werden können.

Zu dem Verfahren a)

Das Verfahren kann ohne zusätzliches Lösungsmittel oder in einem geeigneten Lösungs- oder Dispergiermittel durchgeführt werden. Als Lösungs- oder Dispergiermittel kommen zum Beispiel in Betracht: Aromatische Kohlenwasserstoffe wie zum Beispiel Benzol, Mesitylen, Toluol, Xylol; Pyridin; niedere aliphatische Ketone wie zum Beispiel Aceton, Methyläthylketon; halogenierte Kohlenwasserstoffe wie zum Beispiel Chloroform, 1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol sowie Mischungen der genannten Mittel. Die Reaktion wird beispielsweise bei Temperaturen zwischen 20° bis 200 °C, vorzugsweise 40 bis 160 °C oder auch 50 bis 120 °C durchgeführt. Wird ein Lösungs- beziehungsweise Dispersionsmittel verwendet, arbeitet man häufig bei der Rückflußtemperatur dieses Mittels. Die Reaktion läuft häufig bereits auch schon bei Raumtemperatur ab, beziehungsweise bei einer Temperatur zwischen 40 bis 120 °C.

Die Umsetzung wird vorteilhaft in Gegenwart von säurebindenden Mitteln wie Alkalicarbonaten, Pottasche, Soda, Alkaliacetaten, Alkalihydroxyden oder tertiären Basen (Triethylamin, Pyridin) durchgeführt.

X stellt eine veresterte Hydroxygruppe dar, wobei es sich hierbei um reaktionsfähige Ester handelt Ein reaktionsfähiger Ester ist dabei zum Beispiel derjenige einer starken organischen oder anorganischen Säure, wie vor allem einer Halogenwasserstoffsäure, zum Beispiel der Chlor-, Brom- oder Jodwasserstoffsäure, oder einer Sulfonsäure, wie einer Aryl- oder $C_1$-$C_6$-Alkylsulfonsäure, zum Beispiel von niederen Alkylbenzolsulfonsäuren (p-Toluolsulfonsäure). Als Lösungsmittel kommen insbesondere Mittel wie Dioxan/Wasser, Dimethylformamid/Wasser oder niedere gesättigte aliphatische Alkohole in Frage.

Nicht bekannte Ausgangsstoffe der Formel III können zum Beispiel analog Houben-Weyl, Methoden der Organischen Chemie, Band 5/3 (1962), Seite 503 ff., Band 6/2 (1963), Seite 475 ff. oder Band 9 (1955), Seite 426 erhalten werden.

Zu dem Verfahren b)

Als reaktionsfähige Derivate der Carbonsäure der allgemeinen Formel IV kommen insbesondere die Säurehalogenide (-chloride, -bromide, -jodide), -ester (insbesondere mit $C_1$-$C_6$-Alkanolen) und -anhydri-

de (beispielsweise p-Chlor-benzyliden-phthalid) in Frage. Die Umsetzung wird in An- oder Abwesenheit der üblichen Lösungs- und Hilfsmittel bei Temperaturen zwischen 40 und 200 °C und in weitem pH-Bereich von Saurem bis zum Alkalischen durchgeführt.

Als Lösungsmittel eignen sich zum Beispiel Wasser, aromatische Kohlenwasserstoffe wie zum Beispiel Benzol, Mesitylen, Toluol, Xylol; halogenierte Kohlenwasserstoffe wie zum Beispiel Chloroform, 1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Dimethylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol und Mischungen der genannten Mittel sowie auch tertiäre Amine, zum Beispiel Pyridin. Als Hilfsmittel können in Frage kommen Basen, Säuren und für diese Reaktionen übliche Kondensationsmittel.

Für die Umsetzung von solchen Benzyl-phthalazinon-Derivaten, die erhalten werden, wenn Z der Formel V Wasserstoff ist, mit einer Verbindung Y — Q, kommen ebenfalls die oben angegebenen Lösungsmittel sowie der oben angegebene Temperaturbereich in Frage.

Insbesondere kommen als Lösungsmittel tertiäre Säureamide (zum Beispiel Dimethylformamid), aromatische Kohlenwasserstoffe (zum Beispiel Toluol) oder auch Wasser in Frage, wobei häufig in Gegenwart basischer Stoffe (zum Beispiel Alkalihydroxiden) gearbeitet wird. Vorzugsweise wird bei Temperaturen zwischen 80-200 °C, insbesondere 80-150 °C gearbeitet.

Falls Y der Formel VI ein Halogenatom bedeutet, handelt es sich um Chlor, Brom oder Jod. Falls Y der Formel VI eine Sulfonsäureestergruppe darstellt, handelt es sich beispielsweise um einen $C_1$-$C_6$-Alkylsulfonsäurerest (zum Beispiel $CH_3—SO_2—O—$) oder einen Arylsulfonsäurerest, wie zum Beispiel den Rest einer $C_1$-$C_4$-Alkylbenzolsulfonsäure (zum Beispiel p-Toluolsulfonyloxyrest). Die Benzyl-phthalazinon-Ausgangsverbindung (Verbindung der Formel I, wo sich an dem Säureamid-Stickstoffatom anstelle des Siebenrings mit dem Substituenten R ein Wasserstoffatom befindet) wird beispielsweise auch in Form ihres Alkalisalzes (Na, K) eingesetzt. Derartige Alkalisalze können beispielsweise in üblicher Weise aus dem entsprechenden Phthalazinon und dem Alkalimetall in alkoholischer Lösung (zum Beispiel Ethanol) oder einem anderen hierfür üblichen Mittel zwischen 60 und 100 °C erhalten werden. Die bei der Umsetzung mit Verbindungen der Formel VI erhaltenen Endprodukte stellen gegebenenfalls jeweils Gemische dar aus Verbindungen der Formel I (mit dem 7-Ring) und den entsprechenden Verbindungen, die anstelle des 7-Ringes den Rest

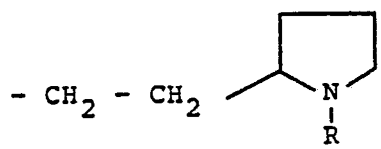

enthalten (Cycloammonium-Umlagerung unter Änderung der Ringgröße). Die Isolierung der Zielverbindung I und von der 5-Ring-Verbindung kann beispielsweise in üblicher Weise durch fraktionierte Kristallisation erfolgen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern, wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Die erfindungsgemäßen Verbindungen der Formel I enthalten ein asymmetrisches Kohlenstoffatom (C-Atom des 7-Ringes, welches mit dem Säureamid-Stickstoffatom des Phthalazinons verbunden ist) und werden daher in der Regel als Racemate erhalten. Solche Racemate können in an sich bekannter Weise beispielsweise durch fraktionierte Kristallisation der Salze von racemischen Verbindungen I mit optisch aktiven Säuren oder auch durch chromatographische Racemattrennung (siehe beispielsweise Angewandte Chemie 92/1 (1980) Seite 14) in die optisch aktiven Isomeren gespalten werden. Es ist aber auch möglich, von vornherein eine optisch aktive Ausgangssubstanz einzusetzen, wobei dann als Endprodukte eine entsprechende optisch aktive Form erhalten wird.

Die vorliegende Erfindung umfasst also die Racemate sowie die entsprechenden optisch aktiven rechts- und linksdrehenden Formen.

Die erfindungsgemäßen Verbindungen sind zur Herstellung Pharmazeutischer Zusammensetzungen beziehungsweise Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel kann unter Verwendung der bekannten und üblichen pharmazeutischen Trägermittel und Hilfsstoffe erfolgen.

Pharmakologische beziehungsweise pharmazeutische Angaben

Die erfindungsgemäßen Verbindungen sind zur Herstellung pharmazeutischer Zusammensetzungen

und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind : Ullmanns Encyklopädie der technischen Chemie, Band 4 (1953), Seite 1 bis 39 ; Journal of Pharmaceutical Sciences, Band 52 (1963), Seite 918 u.ff., H.v. Czetsch-Lindenwald, Hilfsstoffe für Pharmazie und angrenzende Gebiete ; Pharm. Ind., Heft 2, 1961, Seite 72 u.ff. ; Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen und Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie : quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel : Polyacrylsäureester, Celluloseether und ähnliche.

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxyd, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel : Wasser, 1,3-Butandiol, Ethanol, 1,2-Propylenglykol, Polyglykole in Mischung mit Wasser, Ringer's Lösung, isotonische Kochsalzlösung oder auch gehärtete öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage : Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnussöl, Rhizinusöl, Sesamöl, Baumwollsaatöl, Maisöl.

Siehe auch Dr. H.P. Fiedler « Lexikon der Hilfsstoffe für Pharmazie, Kosmetik und angrenzende Gebiete » 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch

verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff (e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80 °C, vorzugsweise 20 bis 50 °C, insbesondere bei Raumtemperatur gearbeitet wird. Im übrigen wird auf das folgende Standardwerk verwiesen : Sucker, Fuchs, Speiser, Pharmazeutische Technologie, Thieme-Verlag Stuttgart, 1978.

Die Applikation der Wirkstoffe beziehungsweise der Arzneimittel kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

Insbesondere ist auch der Zusatz anderer Arzneimittelwirkstoffe möglich beziehungsweise günstig.

Die erfindungsgemäßen Verbindungen zeigen bei der allergischen und nichtallergischen Histamin-Freisetzung an Kaninchen-Leukozyten und Rattenperitonealmastzellen eine gute antiallergische und antihistaminische Wirkung. Die nichtallergische Histamin-Freisetzung wird durch einen Stoff ausgelöst, welcher die Calzium-Kanäle in den Mastzellenmembranen oder Leukozytenmembranen aufmacht und dadurch eine Ausschüttung von Histamin bewirkt (zum Beispiel Ca-Ionophor A 23 187).

Beispielsweise wird bei obengenannter Versuchsmethode bei einer Dosis von 0,3 mg/kg Körpergewicht bei Meerschweinchen eine 50 %ige Hemmung des Asthmaanfalls erhalten.

Diese antiallergische Wirkung ist mit der Wirkung des bekannten Arzneimittels « Azelastine » vergleichbar. Die niedrigste, bereits wirksame Dosis in dem obenangegebenen Tierversuch ist beispielsweise

0,03 mg/kg oral
0,01 mg/kg intravenös.

Als allgemeiner Dosisbereich für die Wirkung (Tierversuch wie oben) kommt beispielsweise in Frage :

0,3-3,0 mg/kg oral
0,1-1,0 mg/kg intravenös.

Indikationen, für die die erfindungsgemäßen Verbindungen in Betracht kommen können : allergisches Asthma, allergische Rhinitis.

Die pharmazeutischen Zubereitungen enthalten im allgemeinen zwischen 0,1 bis 10, vorzugsweise bis 5 mg der erfindungsgemäßen aktiven Komponenten.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen, Als flüssige Anwendungsformen kommen zum Beispiel in Frage : Ölige oder alkoholische beziehungsweise wässrige Lösungen sowie Suspensionen und Emulsionen. Bevorzugte Anwendungsformen sind Tabletten, die zwischen 0,5 und 5 mg oder Lösungen, die zwischen 0,1 bis 3 % an aktiver Substanz enthalten.

Die Einzeldosis der erfindungsgemäßen aktiven Komponenten kann beispielsweise liegen :

a) bei oralen Arzneiformen zwischen 0,5-5 mg, vorzugsweise 2 mg,

b) bei parenteralen Arzneiformen (zum Beispiel intravenös, intramuskulär) zwischen 0,1-1 mg, vorzugsweise 0,5 mg,

c) bei Arzneiformen zur Inhalation (Lösungen oder Aerosole) zwischen 0,5 und 2 mg,

d) bei Arzneiformen zur lokalen Applikation auf die Haut und Schleimhäute (zum Beispiel in Form von Lösungen, Lotionen, Emulsionen, Salben und so weiter) zwischen 1 und 5 mg, vorzugsweise 2 mg.

(Die Dosen sind jeweils bezogen auf die freie Base)

Beispielsweise können 3 mal täglich 1 bis 2 Tabletten mit einem Gehalt von 0,5 bis 5 mg wirksamer Substanz oder zum Beispiel bei intravenöser Injektion 1 bis 2 mal täglich eine Ampulle von 1 bis 2 ml Inhalt mit 0,5 bis 5 mg Substanz empfohlen werden. Die maximale tägliche Dosis bei oraler Verabreichung soll nicht über 10 mg liegen.

Für die Behandlung von Hunden und Katzen liegt die orale Einzeldosis im allgemeinen zwischen ungefähr 0,5 und 5,0 mg/kg Körpergewicht ; die parenterale Dosis ungefähr zwischen 0,3 und 3,0 mg/kg

Körpergewicht.

Für die Behandlung von Pferden und Vieh liegt die orale Einzeldosis im allgemeinen zwischen ungefähr 0,5 und 5,0 mg/kg; die parenterale Einzeldosis ungefähr zwischen 0,3 und 3,0 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation oberhalb 200 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

### Beispiel 1

4-(4-Chlorbenzyl)-2-(hexahydro-1-benzyl-azepin-4-yl)-1-(2H)-phthalazinon

(Formel I, R = Benzyl)

Zu einer auf 50 °C erwärmten Mischung von 6 g (0,013 mol) 4-(4-Chlorbenzyl)-2-(hexahydro-azepin-4-yl)-1-(2H)-phthalazinon x HBr in 60 ml Dioxan werden 3,2 g (0,031 mol = 4,4 ml) Triethylamin und anschließend 1,7 g (0,013 mol = 1,5 ml) Benzylchlorid unter Rühren zugetropft. Nach beendeter Zugabe wird das Reaktionsgemisch 5 Stunden bei 90 °C gerührt. Nach dem Abkühlen werden die ausgefallenen Ammoniumsalze abgesaugt. Die Reaktionslösung wird daraufhin im Vakuum zur Trockene eingeengt. Der Rückstand wird zweimal aus Isopropanol umkristallisiert: Kristalle vom F. 140-141 °C. Ausbeute: 3,1 g (51 %).

Die Ausgangssubstanz wird zum Beispiel wie folgt erhalten:

60 g (0,157 mol) 4-(p-Chlorbenzyl)-2-(hexahydro-1-methylazepin-4-yl)-1-(2H)-phthalazinon werden unter Erwärmung auf 95 °C in 600 ml trockenem Toluol gelöst. Anschließend werden 51,1 g (0,471 mol = 45 ml) Chlorameisensäureethylester in 45 ml Toluol unter Rühren zugetropft. Die Mischung wird 5 Stunden bei 95 °C gerührt. Nach dem Abkühlen auf Raumtemperatur wird das Reaktionsgemisch von Unlöslichem abgesaugt und am Rotationsverdampfer eingeengt. Es verbleibt ein öliger Rückstand, der mit wenig Ether verrieben als weißes kristallines Produkt anfällt und bei 103 bis 105 °C schmilzt. (Ausbeute: 53,4 g (77 %).

53,4 g (0,12 mol) des so erhaltenen 1-Carboxyethyl-Derivats (Formel I, R = $COOC_2H_5$) und 114 ml einer 40 %igen Lösung von Bromwasserstoff in Eisessig werden unter intensivem Rühren 4 Stunden auf 85-90 °C erhitzt; bei zunehmender Erwärmung geht die Carboxyethylverbindung in Lösung. Nach dem Abkühlen wird die Lösung im Vakuum eingeengt. Aus dem erhaltenen zähen, öligen Rückstand erhält man durch Umkristallisation aus Methanol die Ausgangsverbindung der Formel I, worin R Wasserstoff ist, in Form des weißen kristallinen Hydrobromids. Man saugt ab, wäscht mehrfach mit Methanol und trocknet im Vakuum. F. 138-140 °C. Ausbeute: 51 g (95 %).

### Beispiel 2

4-(4-Chlorbenzyl)-2-(hexahydro-1-phenethyl-azepin-4-yl)-1-(2H)-phthalazinon

(Formel I, R = Phenylethyl)

Es wird eine auf 50 °C erwärmte Lösung von 7 g (0,015 mol) 4-(4-Chlorbenzyl)-2-(hexahydro-azepin-4-yl)-1-(2H)-phthalazinon x HBr in 60 ml Dioxan vorgelegt. Anschließend werden 3,8 g (0,037 mol = 5,2 ml) Triethylamin und 2,9 g (0,015 mol) 2-Bromethylbenzol unter Rühren zugetropft und 9 Stunden bei 90 °C gerührt. Nach Abkühlung werden die ausgefallenen Salze abgesaugt und die Lösung am Rotationsverdampfer eingeengt. Der braune ölige Rückstand wird zur Reindarstellung über eine Kieselgelsäule (Elutionsmittel: Diethylether/Methanol = 70 : 30) chromatographiert. Die gewünschten Fraktionen werden vereinigt und das Lösungsmittel im Vakuum abdestilliert. Der Rückstand wird mit 5 ml Isopropanol/HCl und 30 ml Ether versetzt. Das Hydrochlorid fällt daraufhin als klebrige Masse aus. Die überstehende Lösung wird dekantiert, der verbleibende Rückstand in wenig Methylethylketon gelöst und Ether bis zur schwachen Trübung zugegeben. Das über Nacht bei Raumtemperatur auskristallisierte Hydrochlorid wird abgesaugt, mit Methylethylketon ausgewaschen und im Vakuum getrocknet. F. des Hydrochlorids 173-176 °C. Ausbeute: 2,6 g (34 %).

### Beispiel 3

4-(4-Chlorbenzyl)-2-(hexahydro-1-methoxyethyl-azepin-4-yl)-1-(2H)-phthalazinon

(Formel I, R = Methoxyethyl)

6 g (0,013 mol) 4-(4-Chlorbenzyl)-2-(hexahydro-azepin-4-yl)-1-(2H)-phthalazinon x HBr werden zusammen mit 3,6 g (0,026 mol) $K_2CO_3$, 7,4 g (0,078 mol = 7,1 ml) 2-Chlorethylmethylether und 30 ml Dimethylacetamid 2 1/2 Stunden bei einer Ölbadtemperatur von 120 °C gerührt. Nachfolgend wird auf Raumtemperatur abgekühlt und von Unlöslichem abgesaugt. Die Lösung wird im Vakuum eingeengt und das erhaltene bräunliche Öl mittels einer Kieselgelsäule (Elutionsmittel : $CH_2Cl_2/CH_3OH = 90 : 10$) chromatographiert. Die gewünschten Fraktionen werden vereinigt und das Solvens am Rotationsverdampfer abdestilliert. Der ölige Rückstand wird mit Isopropanol/HCl sauer gestellt. Durch Zugabe von Ether wird das Hydrochlorid als klebrige Masse ausgefällt. Nach dem Abdekantieren der überstehenden Lösung wird der Rückstand in der Siedehitze mit Methylethylketon verrieben, wobei das gewünschte Hydrochlorid als kristallines Produkt erhalten wird. F. 194-197 °C. Ausbeute : 1,8 g (28 %).

Beispiel 4

4-(4-Chlorbenzyl)-2-(hexahydro-1-allyl-azepin-4-yl)-1-(2H)-phthalazinon

(Formel I, R = Allyl)

Zu einer auf 50 °C erwärmten Lösung von 6 g (0,013 mol) 4-(4-Chlorbenzyl)-2-(hexahydro-azepin-4-yl)-1-(2H)-phthalazinon x HBr in 60 ml Dioxan gibt man 3,2 g (0,0032 mol = 4,4 ml) Triethylamin und anschließend unter Rühren 1,6 g (0,013 mol = 1,15 ml) Allylbromid. Nach erfolgter Zugabe wird noch 2 Stunden bei einer Temperatur von 60 °C gerührt. Anschließend wird das Reaktionsgemisch filtriert und das Lösungsmittel im Vakuum abdestilliert. Der erhaltene ölige Rückstand wird bei Raumtemperatur in Isopropanol/HCl gelöst und diese Lösung mit Ether bis zur schwachen Trübung versetzt. Über Nacht ist das Hydrochlorid auskristallisiert. Dieses wird abgesaugt, mit Isopropanol nachgewaschen und im Trockenschrank getrocknet. F. des Hydrochlorids 123 °C. Ausbeute : 2,7 g (45 %).

Beispiele für galenische Zubereitungen

Beispiel 1 (Kapseln)

50 g Wirksubstanz gemäß Beispiel 1 werden mit 350 g mikrokristalliner Cellulose, 590 g Milchzucker und 10 g Magnesiumstearat gemischt. Die Mischung wird in einer Menge von jeweils 100 mg in Hartgelatine-Steckkapseln der Größe 3 gefüllt.

Eine Kapsel enthält 5 mg Wirksubstanz.

Beispiel 2 (Tabletten)

50 g Wirksubstanz gemäß Beispiel 1 werden mit 350 g mikrokristalliner Cellulose, 590 g Milchzucker und 10 g Magnesiumstearat gemischt. Diese Mischung wird zu bikonvexen Tabletten vom Gewicht 100 mg, einem Durchmesser von 6 mm und einem Wölbungsradius von 5 mm gepreßt.

Die Tabletten können anschließend nach üblichen Verfahren mit einem magensaftpermeablen oder -löslichen Film überzogen werden.

Beispiel 3 (Ampullen zur Injektion und Inhalation)

10 g Wirksubstanz gemäß Beispiel 1 werden in 400 ml Ethanol gelöst und die Lösung durch Zugabe von Wasser für Injektionszwecke auf 4 Liter aufgefüllt. Die Lösung wird durch ein Membranfilter geeigneter Porenweite steril filtriert. Das Filtrat wird unter aseptischen Bedingungen zu 2 ml in Ampullen abgefüllt. Die Ampullen werden anschließend 20 Minuten lang im gespannten Wasserdampf bei 121 °C sterilisiert.

Eine Ampulle enthält 5 mg Wirksubstanz in 2 ml Lösung.

**Patentansprüche**

1. Substituierte Benzylphthalazinon-Derivate der Formel

(Siehe Formel Seite 8 f.)

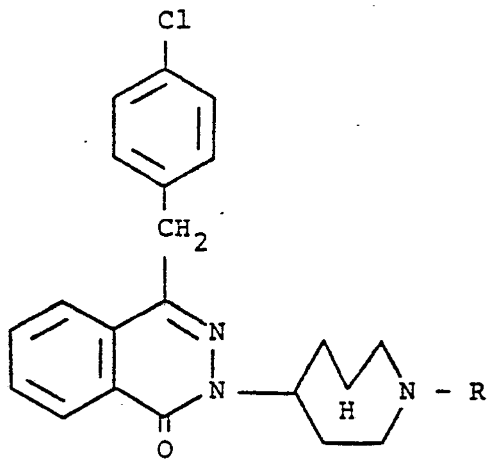

I

worin R ein Benzylrest, ein Phenylethylrest, ein Methoxyethylrest oder ein Allylrest ist und deren physiologisch unbedenkliche Säureadditionssalze.

2. Verfahren zur Herstellung von substituierten Benzylphthalazinon-Derivaten der Formel

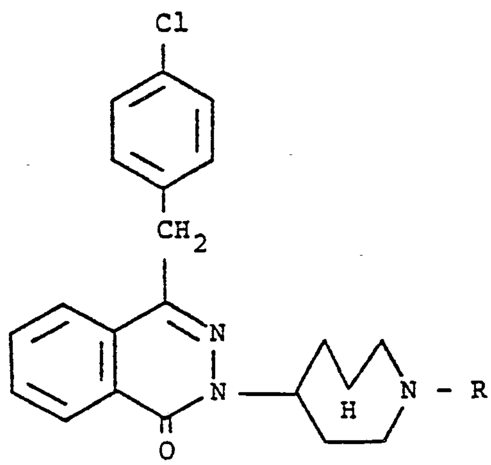

I

worin R ein Benzylrest, ein Phenylethylrest, ein Methoxyethylrest oder ein Allylrest ist und deren physiologisch unbedenklichen Säureadditionssalze, dadurch gekennzeichnet, daß man

a) eine Verbindung der Formel

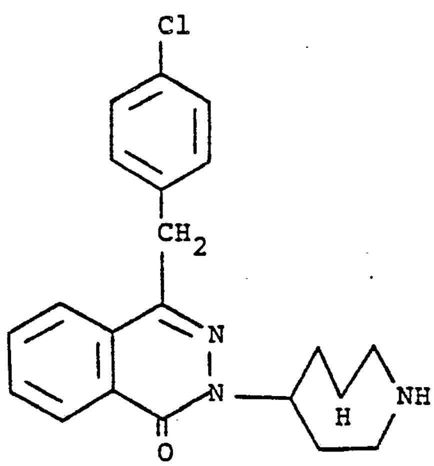

II

mit einer Verbindung der Formel

RX III

umsetzt, wobei R die Bedeutungen wie in Formel I hat und X eine durch eine starke anorganische oder organische Säure veresterte Hydroxygruppe ist oder

b) eine Verbindung der Formel

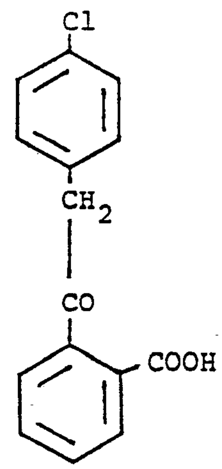

IV

oder ein reaktionsfähiges Derivat derselben mit einem Hydrazin der allgemeinen Formel

$$H_2N{-}NH{-}Z \quad V$$

worin Z Wasserstoff oder der Rest

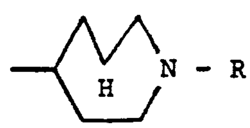

ist und R die Bedeutungen wie in Formel I hat, umsetzt, und in den Fällen, wo Z Wasserstoff ist die erhaltenen Benzyl-phthalazinon-Derivate anschließend mit einer Verbindung der Formel

$$Y{-}Q \quad VI$$

umsetzt, wobei Y ein Halogenatom oder eine Sulfonsäureestergruppe ist und Q entweder den Rest

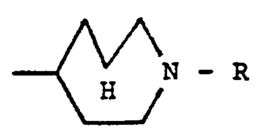

oder den Rest

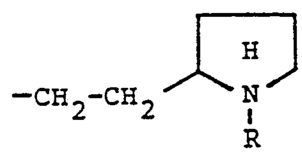

darstellt und R jeweils die bereits angegebenen Bedeutungen hat,
und gegebenenfalls die erhaltenen Verbindungen in ihre Säureadditionssalze überführt.

3. Verbindungen der Formel I zur Anwendung als therapeutische Wirkstoffe.

4. Arzneimittel, enthaltend eine Verbindung der allgemeinen Formel I neben üblichen Träger- und/oder Verdünnungs- beziehungsweise Hilfsstoffen.

5. Verfahren zur Herstellung eines Arzneimittels, dadurch gekennzeichnet, daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I zur Herstellung von Arzneimitteln.

## Claims

1. Substituted benzyl phthalazinone derivatives corresponding to the following formula

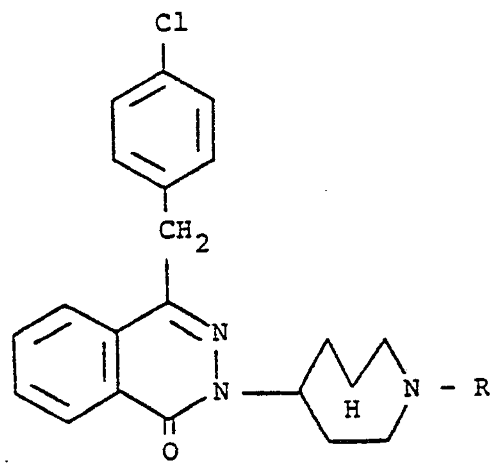

I

in which R is a benzyl radical, a phenylethyl radical, a methoxyethyl radical or an allyl radical, and physiologically acceptable acid addition salts.

2. A process for the preparation of substituted benzyl phthalazinone derivatives corresponding to the following formula

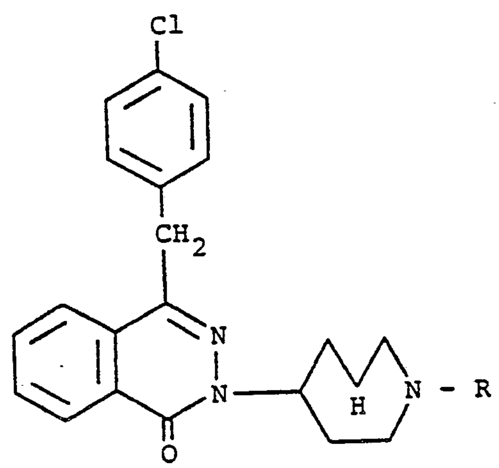

I

in which R is a benzyl radical a phenylethyl radical, a methoxyethyl radical or an allyl radical, and physiologically acceptable acid addition salts thereof, characterized in that

a) a compound corresponding to the following formula

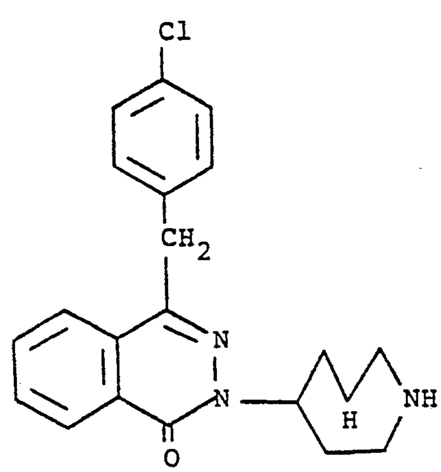

II

is reacted with a compound corresponding to the following formula

RX III

in which R is as defined for formula I and X is a hydroxy group esterified by a strong inorganic or organic acid, or

b) a compound corresponding to the following formula

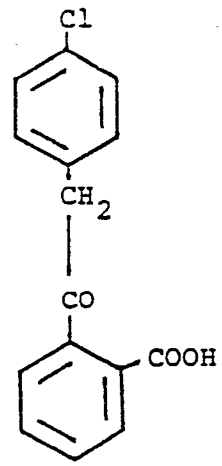

IV

or a reactive derivative thereof is reacted with a hydrazine corresponding to the following general formula

$H_2N—NH—Z$ V

in which Z is hydrogen or the group

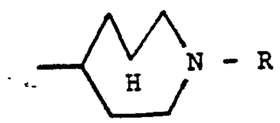

and R is as defined for formula I, and, in cases where Z is hydrogen, the benzyl phthalazinone derivatives obtained are subsequently reacted with a compound corresponding to the following formula

Y—Q VI

in which Y is a halogen atom or a sulfonic acid ester group and Q is either the group

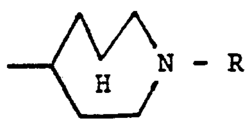

or the group

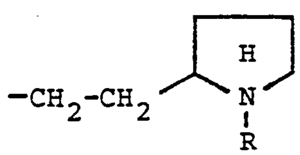

and R is as already defined, and the compounds obtained are optionally converted into their acid addition salts.

3. Compounds corresponding to formula I for use as therapeutic agents.

4. Medicaments containing a compound corresponding to general formula I in addition to typical excipients and/or diluents and auxiliaries.

5. A process for the preparation of a medicament, characterized in that a compound corresponding to general formula I is processed to pharmaceutical preparations or brought into a therapeutically useable form with standard pharmaceutical excipients and/or diluents and other auxiliaries.

6. The use of compounds corresponding to general formula I for the production of medicaments.

**Revendications**

1. Dérivés de benzylphtalazinone substitués de formule

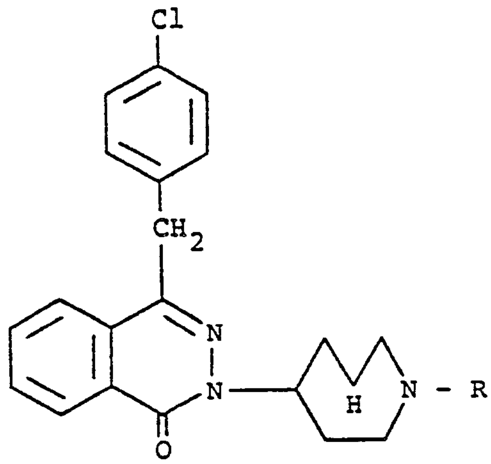

I

dans laquelle R est un radical benzyle, un radical phényléthyle, un radical méthoxyéthyle ou un radical allyle, et leurs sels d'addition d'acides physiologiquement inoffensifs.

2. Procédé de préparation de dérivés de benzylphtalazinone substitués de formule

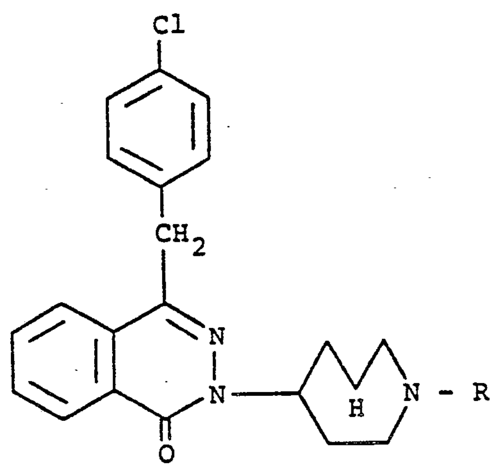

I

dans laquelle R est un radical benzyle, un radical phényléthyle, un radical méthoxyéthyle ou un radical allyle, et de leurs sels d'addition d'acides physiologiquement inoffensifs, caractérisé en ce que

a) l'on fait réagir un composé de formule

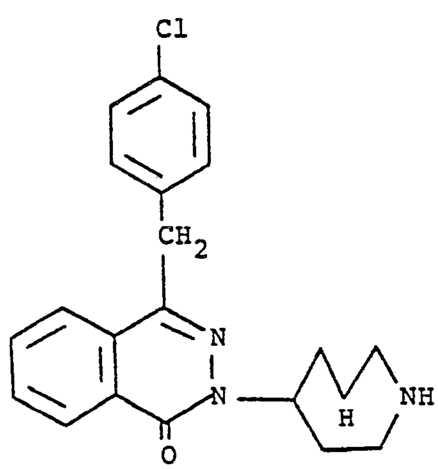

II

avec un composé de formule

RX III

R ayant les mêmes significations que dans la formule I et
X étant un groupement hydroxy estérifié par un acide minéral ou organique fort, ou
b) l'on fait réagir un composé de formule

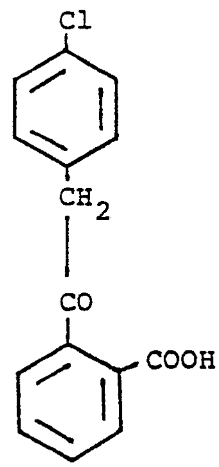

IV

ou un dérivé réactif de celui-ci avec une hydrazine de formule générale

$H_2N—NH—Z$ V

dans laquelle Z est un atome d'hydrogène ou le radical

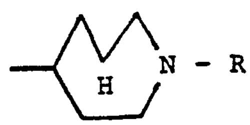

et R a les mêmes significations que dans la formule I et, dans les cas où Z est un atome d'hydrogène, on fait ensuite réagir le dérivé de benzylphtalazinone obtenu avec un composé de formule

Y—Q VI

Y étant un atome d'halogène ou un groupement ester d'acide sulfonique et Q représentant soit le radical

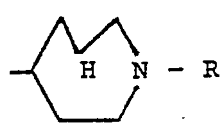

soit le radical

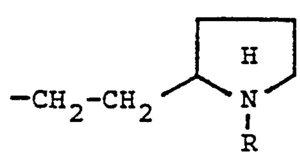

et R ayant les significations déjà indiquées, et, le cas échéant, on transforme les composés obtenus en leurs sels d'addition d'acides.

3. Composés de formule I, destinés à être utilisés comme substances thérapeutiquement actives.

4. Médicament contenant un composé de formule générale I, outre des excipients et/ou diluants ou adjuvants usuels.

5. Procédé de préparation d'un médicament, caractérisé en ce qu'un composé de formule générale I est transformé, avec des excipients et/ou diluants ou autres adjuvants pharmaceutiques habituels, en préparations pharmaceutiques ou mis sous une forme administrable thérapeutiquement.

6. Utilisation de composés de formule générale I pour la préparation de médicaments.